# EUROPEAN PATENT APPLICATION

(11) **EP 4 458 976 A1**
(43) Date of publication of application: **06.11.2024**
(21) Application number: 22913750.0
(22) Date of filing: 28.10.2022
(51) Int. Cl.: C12N 15/864, C07K 14/015, A61K 48/00

(54) **RECOMBINANT ADENO-ASSOCIATED VIRUS (RAAV) GENOME AND SINGLE-POLARITY RAAV VECTOR PACKAGED THEREBY**

(30) Priority: 31.12.2021 CN 202111663496
(71) Applicant: Suzhou Genehealth Biotechnology, Co., Ltd., Kunshan City, Jiangsu 215347 (CN)
(72) Inventor: SHAO, Jiahong, KUNSHAN CITY, Jiangsu 215347 (CN); TAN, Pengcheng, KUNSHAN CITY, Jiangsu 215347 (CN); WU, Xiang, KUNSHAN CITY, Jiangsu 215347 (CN); ZHAO, Xiaoming, KUNSHAN CITY, Jiangsu 215347 (CN); LU, Yang, KUNSHAN CITY, Jiangsu 215347 (CN); XUN, Tingjun, KUNSHAN CITY, Jiangsu 215347 (CN); LEI, Zhenzhen, KUNSHAN CITY, Jiangsu 215347 (CN)
(74) Representative: WSL Patentanwälte Partnerschaft mbB
(86) International application number: PCT/CN2022/128227
(87) International publication number: WO 2023/124487

(57) **Abstract**

Provided are a recombinant adeno-associated virus (rAAV) genome and a single-polarity rAAV (spAAV) vector formed
by packaging same. Specifically, provided is an spAAV genome before packaging, two ends of which are both linear open terminuses,
wherein one end does not have an inverted terminal repeat (ITR) or a part thereof, and the other end has a truncated ITR (ITRT), and
the ITRT does not form a T-shaped hairpin palindromic structure. The spAAV genome is a single-polarity single-stranded DNA. Also
provided are an spAAV vector, a method for delivering an exogenous nucleic acid to a cell by using the vector, and a use of the vector
in the preparation of a product for gene expression, gene therapy, gene editing or gene regulation.

## Description

### Technical Field

The disclosure generally relates to gene carriers and the field of gene therapy, more specifically, to a recombinant adeno-associated virus (rAAV) genome and the single polarity AAV (spAAV) vectors packaged with the same.

### Background

Gene therapy targets patients with genetic mutations or abnormal gene expression causing genetic disorders, including diseases caused by gene defects, dysregulation, or aberrant expression, such as diseases caused by underexpression or overexpression, malignancies, etc. This can be addressed by providing corrective genetic material to treat, prevent, or alleviate these conditions. Currently, gene delivery vectors primarily include viral gene carriers and non-viral gene carriers. Among the many available viral-based gene carriers, such as recombinant retroviruses, recombinant lentiviruses, recombinant adenoviruses, etc., recombinant adeno-associated virus (rAAV) gene carriers are becoming increasingly popular.

Adeno-associated virus (AAV) belongs to the Parvoviridae family and has become one of the most promising gene carriers in gene therapy due to its non-pathogenicity, low immunogenicity, and broad spectrum of infectivity. AAV is an unenveloped single-stranded DNA virus that carries a linear single-stranded DNA genome of approximately 4.7 kb. Each end of the AAV DNA genome contains a 145 bp inverted terminal repeat (ITR), with the 125 bases near the terminal forming a longer palindromic structure that can fold through base pairing, presenting a T-shaped hairpin structure. Sense and antisense strand DNA genomes carrying wild-type inverted terminal repeat are packaged into the AAV viral capsid with equal probability, producing an equal number of sense or antisense strand DNA AAV viral particles. Upon infection, AAV viral particles enter a cell, enter a nucleus, uncoat, and release the AAV genome into the nucleus. The ITRs at the ends of the AAV genome can fold to form a T-shaped hairpin structure, whose 3' end serves as a primer for DNA synthesis to synthesize a second strand, forming a double-stranded DNA molecule, thereby initiating expression of gene carried within the AAV genome. Sense and antisense strand DNA molecules of the AAV genome can also form double-stranded DNA molecules through complementary pairing, expressing genes.

Currently, it is commonly understood in the field that the ITRs at both ends of the AAV DNA genome contain essential information for AAV DNA replication, packaging, integration, and rescue. The rAAV packaging systems used in this field utilize AAV's ITRs as fundamental packaging elements, which are placed at both ends of the exogenous gene DNA to be packaged into the rAAV capsid. Such rAAV vectors have defects in applications such as gene delivery, manipulation, and editing, such as the inability to deliver single-polarity single-stranded DNA for single-strand DNA gene editing; it is challenging to design rAAV vectors for short-term gene expression, such as delivering nucleases for gene editing, which due to prolonged gene expression may cause off-target risks and cytotoxicity; it is difficult to achieve single-strand gene pairing for expressing large genes; and it is not possible to deliver long oligodeoxynucleotides for small nucleic acid gene therapy and gene regulation.

### Summary of the invention

The technical solution provided herein offers a rAAV vector capable of packaging single polarity, single-stranded DNA genomes, also known as single polarity rAAV or spAAV. After extensive research, the inventors have developed an unpackaged spAAV genome that has linear, open-ended termini, with its 5' end containing only a part of the AAV ITR structural sequence elements, and its 3' end containing no AAV ITR structural sequences. The spAAV vector packaged and formed as described herein contains only a single polarity DNA molecule, that is, what's formed is either a sense strand DNA molecule-containing spAAV vector or an antisense strand DNA molecule-containing spAAV vector, not a mixture of both. The spAAV vector can advantageously deliver single-stranded DNA with linear, open-ended termini of the same polarity (i.e., single polarity). That is, the spAAV vector packaged and formed as described herein either delivers sense strand DNA or antisense strand DNA, but not both at the same time, nor are they delivered simultaneously. The spAAV vector packaged and formed as described herein can provide viral particle titers and cell transfection performance comparable to traditional rAAV.

The spAAV vector packaged from the unpackaged spAAV genome described herein will greatly expand the wide applications of AAV vectors in areas such as targeted gene integration, large gene expression, efficient gene expression, short-term gene expression, nuclease-free gene editing, and delivery of long oligodeoxynucleotides, significantly promoting the development and application of AAV gene therapy.

In one aspect, provided herein is an unpackaged spAAV genome, having linear, open-ended termini at both ends, one end lacking ITR or a portion thereof, and the other end comprising ITRt that does not form a T-shaped hairpin palindrome structure.

In another aspect, provided herein is an spAAV vector, comprising, a capsid protein; and an spAAV genome packaged by the capsid protein; wherein the spAAV genome is derived from the unpackaged spAAV genome described herein.

In another aspect, provided herein is an spAAV transgene plasmid for producing the unpackaged spAAV genome described herein.

In another aspect, provided herein is a method for packaging spAAV vectors, comprising, transforming *E. coli* competent cells with the spAAV transgene plasmid described herein, and Cap, Rep expression plasmids; after at least one round of blue-white screening, picking white colonies, amplifying, and extracting recombinant bacmids; using the recombinant bacmids to transfect insect cells to produce recombinant baculovirus; and, extracting the recombinant baculovirus and using it to infect insect cells to obtain spAAV vectors.

In another aspect, provided herein is a method for delivering an exogenous nucleic acid to a cell, comprising contacting the cell with one or more of the spAAV vectors described herein, wherein the one or more of the spAAV vectors comprise the gene expression cassette of the exogenous nucleic acid and/or optionally other DNA sequences.

In another aspect, provided herein is an isolated host cell comprising the spAAV vector described herein.

In another aspect, provided herein is use of the spAAV vector described herein in the preparation of a product for gene expression.

In another aspect, provided herein is use of the spAAV vector described in in the preparation of a product for gene therapy.

In another aspect, provided herein is use of the spAAV vector described herein in the preparation of a product for gene editing.

In another aspect, provided herein is use of the spAAV vector described herein in the preparation of a product for gene regulation.

### Brief description of the drawings

The following description, in conjuction with the figures, futher explains the invention, wherein these figures are solely for illustrative purpose of the embodiments fo the invention, and are not intended to limit the scope of the invention.
Figure 1 shows an exemplary schematic diagram of the DNA sequence structure of AAV2 Flip ITR (containing RBE, trs).
Figure 2 schematically shows the construction and packaging of the spAAV vector according to an exemplary embodiment described herein.
Figure 3 schematically shows the gene expression in cells cotransfected with the sense DNA- and antisense DNA-containing spAAV vectors according to an exemplary embodiment described herein, wherein the sense and antisense DNA of the spAAV vectors pair up.
Figure 4 schematically shows the gene expression involving the integration of the single-stranded DNA of the spAAV vector into the cellular genome according to an exemplary embodiment described herein.
Figure 5 shows an SDS-PAGE electrophoresis image of the spAAV vector capsid proteins. Lane MW: protein molecular weight standard; Lane 1: rAAV-EGFP vector; Lane 2: spAAV-EGFP(+) vector; Lane 3: spAAV-EGFP(-) vector.
Figure 6 shows a neutral agarose gel electrophoresis image of the sense and antisense DNA of the spAAV vectors. Lane MW: DNA molecular weight standard; Lane 1: rAAV-EGFP vector DNA; Lane 2: spAAV-EGFP(+) vector DNA; Lane 3: spAAV-EGFP(-) vector DNA; Lane 4: 2.4kb DNA PCR fragment.
Figure 7 shows an alkaline denaturing agarose gel electrophoresis image of the sense and antisense DNA of the spAAV vectors. Lane MW: DNA molecular weight standard; Lane 1: rAAV-EGFP vector DNA; Lane 2: spAAV-EGFP(+) vector DNA; Lane 3: spAAV-EGFP(-) vector DNA; Lane 4: 2.4kb DNA PCR fragment.
Figure 8 shows a neutral agarose gel electrophoresis image of the sense and antisense DNA of the spAAV vectors and their pairing. Lane MW: DNA molecular weight standard; Lane 1: rAAV-EGFP vector DNA; Lane 2: spAAV-EGFP(+) vector DNA; Lane 3: spAAV-EGFP(-) vector DNA; Lane 4: annealed complementary products of spAAV-EGFP(+) vector DNA + spAAV-EGFP(-) vector DNA; Lane 5: 2.4kb DNA PCR fragment.
Figure 9 shows a neutral agarose gel electrophoresis image of the sense and antisense DNA of the spAAV vectors and their pairing, after digestion with S1 nuclease. Lane MW: DNA molecular weight standard; Lane 1: rAAV-Gli1 vector DNA; Lane 2: spAAV-EGFP(+) vector DNA; Lane 3: spAAV-EGFP(-) vector DNA; Lane 4: spAAV-EGFP(+) vector DNA + spAAV-EGFP(-) vector DNA; Lane 5: 2.4kb DNA PCR fragment; Lane 6: denatured rAAV-Gli1 vector DNA; Lane 7: denatured spAAV-EGFP(+) vector DNA + spAAV-EGFP(-) vector DNA; Lane 8: denatured 2.4kb DNA PCR fragment.
Figure 10 shows fluorescent images of gene expression in HEK293 cells separately and co-infected with the sense and antisense strand spAAV vectors. A. Virus vector infecting HEK293, 24 hours, EGFP expression; B. Virus vector infecting HEK293, 3 days, EGFP expression; C. Virus vector infecting HEK293, 7 days, EGFP expression; D. Virus vector infecting HEK293, 14 days, EGFP expression. 1. rAAV-EGFP; 2. spAAV-EGFP(+); 3. spAAV-EGFP(-); 4. Co-infection of spAAV-EGFP(+) and spAAV-EGFP(-).

### Detailed description

The meanings of the technical terms used in the disclosure are consistent with the general understanding of those skilled in the relevant art, unless otherwise specified. In the disclosure, the term "one" or its combinations with various quantifiers encompasses both singular and plural meanings, unless specifically stated otherwise. In the disclosure, for the same parameter or variable, when multiple values, value ranges, or combinations thereof are provided, it is equivalent to specifically disclosing these values, range endpoints, and the value ranges that can be formed by any combination of them. In the disclosure, any value, whether modified by terms like "about" or not, covers an approximate range understandable to those skilled in the art, such as ±10%, ±5%, etc. Each "embodiment" described herein equally refers to and encompasses the embodiments of all methods and systems of the disclosure. In the disclosure, one or more technical features in any embodiment can be freely combined with one or more technical features from any other embodiment, and the resulting embodiments are also disclosed in the disclosure.

Adeno-associated virus (AAV) is a member of the Parvoviridae family. It is non-enveloped and has an icosahedral symmetry structure. Currently, more than a dozen serotypes and over a hundred variants of AAV have been found, among which AAV2 is the most thoroughly studied and widely used serotype.

The AAV genome includes approximately 4.7 kb of linear single-stranded DNA, with ITRs of about 145 bases at both ends. About 125 bases near the ends of the ITRs contain palindromic sequences, which can be folded through base-pairing, presenting a T-shaped hairpin structure. The ITRs comprise Rep binding site (RBE) and terminal resolution sites (trs), which can be recognized and bound by Rep proteins, and a nick is produced at the trs.

Between the ITRs at both ends of the AAV genome, there are two open reading frames (ORFs) that encode for rep and cap, respectively. The rep gene encodes four Rep proteins: Rep78, Rep68, Rep52, and Rep40, which play roles in the replication, integration, rescue, and packaging of the AAV virus. The cap gene encodes the AAV virus capsid proteins VP1, VP2, and VP3, where VP1 is essential for forming infectious AAV, and VP3 is the main protein that constitutes the AAV virion particles. The ratio of VP1, VP2, and VP3 in the AAV virus capsid is approximately 1:1:10.

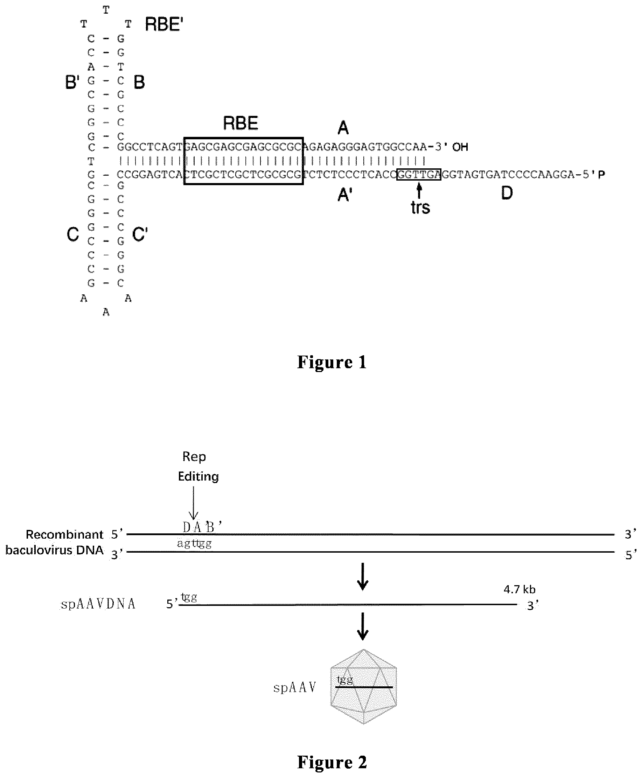

Figure 1 shows a schematic diagram of the DNA sequence structure of AAV2 Flip ITR, where the ITR forms a T-shaped palindromic hairpin structure. As shown in Figure 1, the first 125 bases of the ITR sequence can be divided into different sections A', C', C, B', B, and A, where B is reversely complementary to B' and C is reverse complementary to C' to form the T-shaped palindromic hairpin structure, and A and A' forms the stem of the hairpin. Following the A' section, there is also a D section. The positions of RBE and trs are illustrated in Figure 1.

Although the ITR sequences vary among different serotypes of AAV genomes, they all can form a T-shaped palindromic hairpin structure and contain RBE and trs.

During the assembly of AAV to form virus particles, sense and antisense strand DNA genomes containing the wild-type ITRs are packaged into the AAV capsid with equal probability, thereby producing an equal number of sense and antisense AAV virus particles. Once the AAV virus particles infect and enter a cell, they uncoat and release the AAV genome within the cell. The ITRs at the ends of the AAV genome fold to form a T-shaped palindromic hairpin structure, and use the 3' end as a primer, synthesize the second strand to form a double-stranded molecule, thereby initiating the expression of genes carried by the AAV genome. The sense and antisense DNA molecules of the AAV genome can also form double-stranded DNA through complementary pairing, leading to gene expression.

In one aspect, provided herein is an unpackaged spAAV genome, having linear, open-ended termini at both ends, one end lacking ITR or a portion thereof, and the other end comprising ITRt that does not form a T-shaped hairpin palindrome structure.

As used herein, the term "single polarity" or "sp" refers to a single DNA polarity, i.e., an spAAV vector produced by packaging the unpackaged spAAV genome described herein with only one polarity, either an spAAV vector with sense strand DNA or an spAAV vector with antisense strand DNA, but not both simultaneously. The spAAV vector produced by packaging the unpackaged spAAV genome described herein is also referred to as a "single polarity rAAV vector" or "spAAV vector."

As used herein, the term "recombinant adeno-associated virus vector" or "rAAV" refers to a non-wild-type adeno-associated virus particle used as a gene delivery vector and comprising a recombinant AAV genome packaged within a viral capsid.

As used herein, the term "unpackaged spAAV genome" refers to the recombinant AAV genome before packaging or to be packaged, which is intended for subsequent packaging into capsid proteins.

As used herein, the term "open-ended" or "linear open-ended" refers to the 3' or 5' nucleotide sequence ends that do not have any other structure (e.g., a hairpin structure) except for a linear straight chain.

As used herein, the term "ITR or a portion thereof' refers to all or part of the DNA segments of the ITR, which include "ITR elements". For example, in the case of Flip ITR (e.g., as shown in Figure 1), ITR elements include but are not limited to: D region, trs site, RBE site, A' region (part), C' region, C region, B' region, B region, A region, etc.

It should be understood that the ITR sequences present at the ends of the AAV genome may have the same or different segment orientations, such as Flip/Flop, Flop/Flip, Flip/Flip, or Flop/Flop. An exemplary structural orientation of "Flip" ITR is shown in Figure 1. An exemplary structural orientation of "Flop" ITR is shown in Figure 1, except that the positions of the B' region and the C' region are swapped, and the positions of the B region and the C region are swapped.

As used herein, the term "ITR truncated portion" or "ITRt" differs from the ITR that can form a T-shaped hairpin palindrome structure. It refers to the portion of the DNA segment of the ITR derived from the ITR that does not form a hairpin palindrome structure (e.g., partial ITR elements), forming a linear open-ended end, and allowing the rAAV genome to be packaged.

As used herein, the term "hairpin structure" or "hairpin loop structure" refers to the "hairpin" shape formed by the DNA molecule folding back on itself, bringing certain bases close together within the folded region and forming complementary pairs, such as a T-shaped hairpin structure, e.g., as shown in Figure 1.

Unlike traditional unpackaged rAAV genomes, the unpackaged spAAV genome provided herein does not comprise ITRs that can form hairpin structures (e.g., T-shaped hairpin structures) at both ends of its linear single-stranded DNA. Instead, it has an ITRt at the 5' end that forms a linear open-ended structure without forming a hairpin structure. Surprisingly, the inventors have discovered that the spAAV vector formed by packaging the unpackaged spAAV genome described herein has only one polarity, either a sense strand DNA spAAV vector or an antisense strand DNA spAAV vector, but not both simultaneously. Additionally, the titer and cell transfection performance of the spAAV viral particles produced by packaging are comparable to traditional rAAV viral particles. Using the spAAV viral particles formed by packaging described herein, it is possible to advantageously deliver single polarity, single-stranded DNA with a linear open-ended structure. Because it is single polarity, single-stranded DNA with no 3' ITR structure that can be used as a primer for DNA replication, spAAV DNA can only exist in the cell as single-stranded DNA. Thus, it can be used for gene integration, gene editing, efficient DNA pairing to express large genes, and delivery of long oligodeoxynucleotides for small nucleic acid gene therapy, etc.

In some embodiments, the spAAV genome is single-stranded DNA.

In some embodiments, the unpackaged spAAV genome does not comprise ITRs or portions thereof at the 3' end. In some embodiments, the unpackaged spAAV genome comprises the ITRt at the 5' end. In some embodiments, the unpackaged spAAV genome does not comprise ITRs or portions thereof at the 3' end and comprises the ITRt at the 5' end.

Since the sequence at the 3' end does not comprise an ITR palindrome structure, the resulting spAAV vector cannot synthesize a second strand using the 3' end as a primer to form a double-stranded DNA molecule after entering the host cell.

In some embodiments, the ITRt comprises trs and RBE. In some embodiments, the ITRt comprises the D, A', and C' segments derived from the Flip ITR, such as the corresponding structure shown in Figure 1. In some embodiments, the ITRt comprises the D, A', and B' segments derived from the Flop ITR, etc.

In some exemplary embodiments, the unpackaged spAAV genome comprises, in the 5' to 3' order: ITRt, exogenous gene expression cassette, and/or optionally other exogenous DNA sequences, wherein the ITRt comprises RBE and trs. In some exemplary embodiments, the unpackaged spAAV genome comprises, in the 5' to 3' order: ITRt, exogenous gene expression cassette, and/or optionally other exogenous DNA sequences, wherein the ITRt comprises the D, A', and C' segments derived from the Flip ITR. In some exemplary embodiments, the unpackaged spAAV genome comprises, in the 5' to 3' order: ITRt, exogenous gene expression cassette, and/or optionally other exogenous DNA sequences, wherein the ITRt comprises the D, A', and B' segments derived from the Flop ITR.

In some exemplary embodiments, the ITR described herein has the following nucleotide sequence (Flip ITR):
Flip ITR (SEQ ID NO: 1):

aggaacccctagtgatggagttggccactccctctctgcgcgctcgctcgctcactgaggccgcccgg
gcaaagcccgggcgtcgggcgacctttggtcgcccggcctcagtgagcgagcgagcgcgcagagagggagtgg
ccaa

In some exemplary embodiments, the ITR described herein has the following nucleotide sequence (Flop ITR):
Flop ITR (SEQ ID NO: 2):

aggaacccctagtgatggagttggccactccctctctgcgcgctcgctcgctcactgaggccgggcga
ccaaaggtcgcccgacgcccgggctttgcccgggcggcctcagtgagcgagcgagcgcgcagagagggagtgg
ccaa

In some exemplary embodiments, the nucleotide sequence of the ITR described herein has greater than 80%, greater than 85%, greater than 90%, greater than 95%, greater than 98%, greater than 99%, or 100% sequence identity to the nucleotide sequence shown in SEQ ID NO: 1 or SEQ ID NO: 2. In some exemplary embodiments, the nucleotide sequence of the ITR described herein is the nucleotide sequence shown in SEQ ID NO: 1 or SEQ ID NO: 2.

In some exemplary embodiments, the nucleotide sequence of the RBE described herein has the nucleotide sequence shown in SEQ ID NO: 3:
RBE (SEQ ID NO: 3):
gcgc gctc gctc gctc

In some exemplary embodiments, the nucleotide sequence of the RBE described herein has greater than 80%, greater than 85%, greater than 90%, greater than 95%, greater than 98%, greater than 99%, or 100% sequence identity to the nucleotide sequence shown in SEQ ID NO: 3. In some exemplary embodiments, the nucleotide sequence of the RBE described herein is the nucleotide sequence shown in SEQ ID NO: 3.

In some exemplary embodiments, the nucleotide sequence of the trs described herein has the nucleotide sequence shown in SEQ ID NO: 4:
trs (SEQ ID NO: 4):
agttgg

In some exemplary embodiments, the nucleotide sequence of the trs described herein has greater than 80%, greater than 85%, greater than 90%, greater than 95%, greater than 98%, greater than 99%, or 100% sequence identity to the nucleotide sequence shown in SEQ ID NO: 4. In some exemplary embodiments, the nucleotide sequence of the trs described herein is the nucleotide sequence shown in SEQ ID NO: 4.

In some exemplary embodiments, the ITRt described herein has the nucleotide sequence shown in SEQ ID NO: 5:
ITRt (SEQ ID NO: 5):
aggaacccctagtgatggagttggccactccctctctgcgcgctcgctcgctcactgaggccgggcgaccaaa

In some exemplary embodiments, the nucleotide sequence of the ITRt described herein has greater than 80%, greater than 85%, greater than 90%, greater than 95%, greater than 98%, greater than 99%, or 100% sequence identity to the nucleotide sequence shown in SEQ ID NO: 5. In some exemplary embodiments, the nucleotide sequence of the ITRt described herein is the nucleotide sequence shown in SEQ ID NO: 5.

It will be understood by those skilled in the art that the ITR, RBE, and trs sequences described above are provided for exemplary, non-limiting purposes, based on AAV2. Those skilled in the art are able to determine the ITR sequences and RBE, trs sequences of various types (e.g., serotypes) of rAAV and implement the invention with appropriate adjustments without departing from the teachings herein.

In some embodiments, the unpackaged spAAV genome does not comprise nucleotide sequences encoding the rep gene and/or cap gene.

In some embodiments, the unpackaged spAAV genome comprises exogenous nucleic acids, such as an exogenous gene expression cassette, between the end with ITRt and the other end. The exogenous nucleic acids may comprise nucleotide sequences for encoding an exogenous gene. In some embodiments, the unpackaged spAAV genome comprises optional DNA sequences between the end with ITRt and the other end. The optional DNA sequences may be nucleotide sequences that do not encode an exogenous gene, such as filler sequences.

As used herein, the terms "exogenous gene," "exogenous nucleic acid," or "target gene" refer to genes or DNA originating from outside the organism of interest or under study (e.g., organism individual). The exogenous gene, exogenous nucleic acid, or target gene may be any exogenous gene or exogenous DNA that is desired to be expressed or to produce a biological function in the recipient cells to which the spAAV viral particles are delivered.

In some embodiments, the spAAV genome comprises a sense strand single-stranded DNA sequence of exogenous nucleic acids (e.g., exogenous gene expression cassette). In some embodiments, the spAAV genome comprises an antisense strand single-stranded DNA sequence of exogenous nucleic acids (e.g., exogenous gene expression cassette).

In some embodiments, the nucleotide sequence for encoding the exogenous gene comprises a forward exogenous gene expression cassette. In some exemplary embodiments, the forward exogenous gene expression cassette may comprise, in the 5'-3' direction, a promoter, an exogenous gene open reading frame, and a polyA sequence. In some exemplary embodiments, the forward exogenous gene expression cassette may further comprise an enhancer, an intron. In some exemplary embodiments, the enhancer and intron are located between the promoter and the exogenous gene open reading frame. In some exemplary embodiments, the forward exogenous gene expression cassette may comprise, in the 5'-3' direction, DNA sequences for gene editing and gene regulation.

In some embodiments, the nucleotide sequence for encoding the exogenous gene comprises a reverse exogenous gene expression cassette. In some exemplary embodiments, the reverse exogenous gene expression cassette may comprise, in the 5'-3' direction, a reverse complementary sequence of the polyA sequence, a reverse complementary sequence of the exogenous gene open reading frame, and a reverse complementary sequence of the promoter. In some exemplary embodiments, the reverse exogenous gene expression cassette may further comprise an enhancer, an intron. In some exemplary embodiments, the enhancer and intron are located between the reverse complementary sequence of the promoter and the reverse complementary sequence of the exogenous gene open reading frame. In some exemplary embodiments, the reverse exogenous gene expression cassette may comprise, in the 5'-3' direction, reverse complementary sequences of DNA sequences for gene editing and gene regulation.

In some embodiments, the promoter may include, for example, a CMV promoter, a CAG promoter, or a cell-specific promoter, such as a GFAP promoter, hAAT promoter, etc.

In some embodiments, the unpackaged spAAV genome can be produced by constructing a plasmid (e.g., spAAV transgene plasmid). The spAAV transgene plasmid described herein can be any plasmid suitable for producing the unpackaged spAAV genome described herein. Suitable spAAV transgene plasmids can be based on, for example, but not limited to, pFastBacdual, pFastBac1 plasmids, etc.

The unpackaged spAAV genome can subsequently be packaged into capsid proteins by an spAAV vector packaging system to form spAAV vectors. In some embodiments, the spAAV vector packaging system may include, for example, insect cell baculovirus packaging system, mammalian cell packaging system, etc.

In another aspect, provided herein is an spAAV vector, comprising,
a capsid protein; and
an spAAV genome packaged by the capsid protein;
wherein the packaged spAAV genome is derived from the unpackaged spAAV genome described herein.

As used herein, "spAAV vector" is also referred to as "spAAV viral particles," which are produced by packaging the unpackaged spAAV genome into AAV capsid proteins (e.g., using an spAAV vector packaging system).

In some embodiments, examples of the spAAV vector packaging system may include, for example, an insect cell baculovirus packaging system, a mammalian cell packaging system, etc.

In some embodiments, the spAAV genome packaged into capsid proteins is single polarity, single-stranded DNA.

In some embodiments, the spAAV genome packaged into capsid proteins (also referred to herein as "packaged spAAV genome") has linear open-ended termini at both ends, with one end not comprising ITR or portions thereof and the other end comprising an ITRt-derived sequence.

As used herein, the term "ITRt-derived sequence" is obtained by cutting at the trs site contained in the ITRt, forming a linear open-ended terminus without forming a hairpin structure.

In some embodiments, the packaged spAAV genome has an ITRt-derived sequence at the 5' end. In some embodiments, the packaged spAAV genome does not comprise ITR or portions thereof at the 3' end. In some embodiments, the packaged spAAV genome has an ITRt-derived sequence at the 5' end and does not comprise ITR or portions thereof at the 3' end.

In some embodiments, the ITRt-derived sequence comprises RBE. In some embodiments, the ITRt-derived sequence comprises RBE and portions of the trs sequence (remaining trs portions after Rep cutting).

In some embodiments, the packaged spAAV genome does not comprise nucleotide sequences encoding the rep gene and/or cap gene.

In some embodiments, the packaged spAAV genome comprises exogenous nucleic acids, such as an exogenous gene expression cassette, between the end with the ITRt-derived sequence and the other end. The exogenous nucleic acids may comprise nucleotide sequences for encoding an exogenous gene. In some embodiments, the nucleotide sequence for encoding the exogenous gene is a single polarity, single-stranded DNA sequence. In some embodiments, the packaged spAAV genome comprises optional DNA sequences between the end with the ITRt-derived sequence and the other end. The optional DNA sequences may be nucleotide sequences that do not encode an exogenous gene, such as filler sequences.

In some embodiments, the packaged spAAV genome comprises sense strand single-stranded DNA sequences of exogenous nucleic acids (e.g., exogenous gene expression cassette). In some embodiments, the packaged spAAV genome comprises antisense strand single-stranded DNA sequences of exogenous nucleic acids (e.g., exogenous gene expression cassette).

In some embodiments, the nucleotide sequence for encoding the exogenous gene comprises a forward exogenous gene expression cassette. In some exemplary embodiments, the forward exogenous gene expression cassette may comprise, in the 5'-3' direction, a promoter, an exogenous gene open reading frame, and a polyA sequence. In some exemplary embodiments, the forward exogenous gene expression cassette may further comprise an enhancer and an intron. In some exemplary embodiments, the enhancer and intron are located between the promoter and the exogenous gene open reading frame. In some exemplary embodiments, the forward exogenous gene expression cassette may comprise, in the 5'-3' direction, DNA sequences for gene editing and gene regulation.

In some embodiments, the nucleotide sequence for encoding the exogenous gene comprises a reverse exogenous gene expression cassette. In some exemplary embodiments, the reverse exogenous gene expression cassette may comprise, in the 5'-3' direction, a reverse complementary sequence of the polyA sequence, a reverse complementary sequence of the exogenous gene open reading frame, and a reverse complementary sequence of the promoter. In some exemplary embodiments, the reverse exogenous gene expression cassette may further comprise an enhancer and an intron. In some exemplary embodiments, the enhancer and intron are located between the reverse complementary sequence of the promoter and the reverse complementary sequence of the exogenous gene open reading frame. In some exemplary embodiments, the reverse exogenous gene expression cassette may comprise, in the 5'-3' direction, reverse complementary sequences of DNA sequences for gene editing and gene regulation.

In some embodiments, the promoter may include, for example, a CMV promoter, a CAG promoter, or a cell-specific promoter, such as a GFAP promoter, hAAT promoter, etc.

In some embodiments, the capsid proteins are AAV capsid proteins. In some embodiments, the capsid proteins can be selected from capsid proteins of various AAV types (AAV1-AAV12, including AAV1, AAV2, AAV3, AAV4, AAV5, AAV6, AAV7, AAV8, AAV9, AAV10, AAV11, and AAV12).

In some embodiments, the capsid proteins are variants of AAV capsid proteins, such as tyrosine single amino acid/multiple amino acid variants, tyrosine, serine, threonine variants, or multiple serotype chimeras like AAV-DJ, or polypeptide insertion variants.

In some embodiments, the capsid proteins can be produced by using an spAAV vector packaging system from a plasmid expressing AAV capsid protein Cap.

In some embodiments, the spAAV vector can be produced by packaging the spAAV transgene plasmid (which produces the unpackaged spAAV genome) and the plasmid expressing AAV capsid protein Cap and replication protein Rep (which produces Cap and Rep proteins) using an spAAV vector packaging system. In some embodiments, the AAV Cap encoding gene and the AAV Rep encoding gene can be on the same plasmid or on two different plasmids. In some embodiments, the AAV Cap and/or AAV Rep expression plasmid can include Cap and/or Rep gene expression cassettes and necessary expression elements, as well as intron sequences to enhance expression. There are no special restrictions on the plasmid used to express AAV Cap and/or Rep, and those skilled in the art can routinely apply AAV Cap and/or Rep expression plasmids, see, for example, Urabe M, Ding C, Kotin RM. Insect cells as a factory to produce adeno-associated virus type 2 vectors. Hum Gene Ther. 2002 Nov 1;13(16):1935-43. doi: 10.1089/10430340260355347. PMID: 12427305.

In another aspect, provided herein is an spAAV transgene plasmid for producing the unpackaged spAAV genome described herein.

The spAAV transgene plasmid described herein can be any plasmid suitable for producing the unpackaged spAAV genome described herein. Suitable spAAV transgene plasmids can be based on, for example but not limited to, pFastBacdual plasmid, pFastBac 1 plasmid, etc.

In some exemplary embodiments, the spAAV transgene plasmid can be designed to encode a sense strand single-stranded DNA sequence of an exogenous gene, which may comprise, in the 5' to 3' order: ITRt (comprising RBE and trs), promoter (such as CMV), exogenous gene open reading frame, and polyA. In some exemplary embodiments, the spAAV transgene plasmid may further comprise filler sequences. In some specific embodiments, the filler sequences may be located, for example, upstream of the promoter and/or downstream of the polyA.

In some exemplary embodiments, the spAAV transgene plasmid can be designed to encode an antisense strand single-stranded DNA sequence of an exogenous gene, which may comprise, in the 5' to 3' order: ITRt (comprising RBE and trs), polyA', exogenous gene open reading frame', and promoter' (such as CMV'). In some exemplary embodiments, the spAAV transgene plasmid may further comprise filler sequences. In some specific embodiments, the filler sequences may be located, for example, downstream of the promoter' and/or upstream of the polyA'.

As used herein, the term "filler sequence" refers to auxiliary DNA added to increase the DNA length to reach the AAV packaging length of 4.7 kb in cases where the length of some target DNA is less than the AAV packaging length. In some embodiments, the filler sequence may have various lengths as long as the final DNA reaches a length of about 4.7 kb, for example.

The spAAV transgene plasmid can be packaged together with the Rep and Cap gene expression plasmids using an spAAV vector packaging system to produce the spAAV vector described herein.

In another aspect, provided herein is a method for packaging spAAV vectors, comprising, transforming *E. coli* competent cells with the spAAV transgene plasmid described herein, and Cap, Rep expression plasmids; after at least one round of blue-white screening, picking white colonies, amplifying, and extracting recombinant bacmids; using the recombinant bacmids to transfect insect cells to produce recombinant baculovirus; and, extracting the recombinant baculovirus and using it to infect insect cells to obtain spAAV vectors.

In some embodiments, the competent cells of E. coli are E. coli DH10Bac competent cells. In some embodiments, the spAAV vector packaging system is an insect cell baculovirus packaging system.

In some embodiments, after at least one round of blue-white screening, white colonies are picked, amplified, and recombinant bacmids are extracted. In some embodiments, after two or more rounds of blue-white screening, white colonies are picked, amplified, and recombinant bacmids are extracted.

In some embodiments, the recombinant bacmids are transfected into insect cells (e.g., insect cells Sf9) to produce recombinant baculoviruses (e.g., P3 generation recombinant baculoviruses).

In some embodiments, recombinant baculoviruses (e.g., two or three types of P3 generation recombinant baculoviruses) are used to infect (e.g., co-infect) insect cells (e.g., Sf9 insect cells) to package and obtain spAAV vectors.

Figure 2 shows a schematic diagram of spAAV vector construction and packaging according to an exemplary embodiment described herein. In this method, during the packaging of the spAAV vector, the Rep protein recognizes the Rep binding site (RBE) on the recombinant baculovirus DNA, binds to this site, identifies the terminal resolution site (trs), cleaves the phosphodiester bond between the bases tt, covalently binds to the 5' end, introduces the DNA into the AAV capsid for packaging, with the packaged DNA length being approximately 4.7 kb.

For the method of packaging rAAV vectors using an insect cell baculovirus packaging system, see also, for example, Urabe M, Ding C, Kotin RM. Insect cells as a factory to produce adeno-associated virus type 2 vectors. Hum Gene Ther. 2002 Nov 1; 13(16):1935-43. doi: 10.1089/10430340260355347. PMID: 1242730.

In another aspect, provided herein is a method for delivering an exogenous nucleic acid to a cell, comprising contacting the cell with one or more of the spAAV vectors described herein, wherein the one or more of the spAAV vectors comprise the gene expression cassette of the exogenous nucleic acid and/or optionally other DNA sequences.

In some embodiments, the cells can be eukaryotic cells. In some embodiments, the cells can be animal cells. In some embodiments, the cells can be vertebrate cells. In some embodiments, the cells can be mammalian cells, such as human cells.

In some embodiments, the exogenous nucleic acid comprises the nucleotide sequence of an exogenous gene. In some embodiments, the exogenous nucleic acid comprises a nucleotide sequence that is not of an exogenous gene.

In some embodiments, the genome of the spAAV vector comprises the sense strand single-stranded DNA sequence or the antisense strand single-stranded DNA sequence of the exogenous gene.

In some embodiments, one or more of the spAAV vectors comprise an spAAV vector containing only the sense strand single-stranded DNA sequence of the exogenous nucleic acid and/or an spAAV vector containing only the antisense strand single-stranded DNA sequence of the exogenous nucleic acid.

In some embodiments, one or more of the spAAV vectors comprise two or more spAAV vectors, which include at least a first spAAV vector having the sense strand single-stranded DNA sequence of the exogenous gene and a second spAAV vector having the antisense strand single-stranded DNA sequence of the exogenous gene.

In some embodiments, cells are contacted with the spAAV vector having the sense strand single-stranded DNA sequence of the exogenous gene. In some embodiments, cells are contacted with the spAAV vector having the antisense strand single-stranded DNA sequence of the exogenous gene. In a preferred embodiment, cells are contacted with both the first spAAV vector having the sense strand single-stranded DNA sequence of the exogenous gene and the second spAAV vector having the antisense strand single-stranded DNA sequence of the exogenous gene. In some embodiments, cells are simultaneously contacted with both the first spAAV vector having the sense strand single-stranded DNA sequence of the exogenous gene and the second spAAV vector having the antisense strand single-stranded DNA sequence of the exogenous gene. In some embodiments, cells are sequentially (e.g., within 24 hours) contacted with the first spAAV vector having the sense strand single-stranded DNA sequence of the exogenous gene and the second spAAV vector having the antisense strand single-stranded DNA sequence of the exogenous gene, or vice versa. It should be understood that "first spAAV vector" and "second spAAV vector" are used herein only to distinguish the two and are not intended to limit the order of use.

When cells are infected with the spAAV vector having the sense strand single-stranded DNA sequence of the exogenous gene, or the spAAV vector having the antisense strand single-stranded DNA sequence of the exogenous gene, the exogenous gene carried by the vector has a higher probability (e.g., 20%) of being integrated into the cell genome, resulting in long-term and stable expression of the exogenous gene.

By jointly using the spAAV vector having the sense strand single-stranded DNA sequence of the exogenous gene and the spAAV vector having the antisense strand single-stranded DNA sequence of the exogenous gene, it facilitates the rapid complementary pairing of the sense strand and antisense strand spAAV genome in the host cell nucleus to form a double-stranded molecule, thereby initiating gene expression.

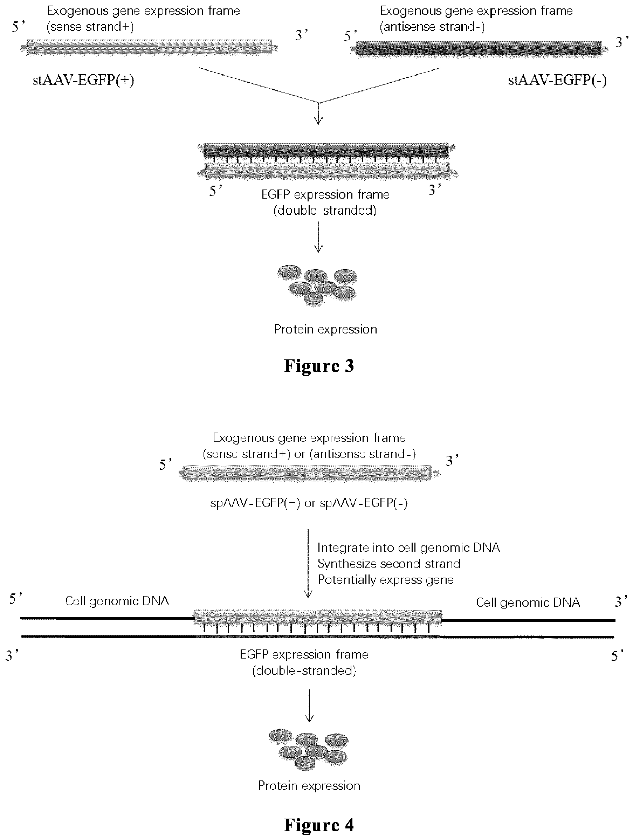

Figure 3 shows a schematic diagram of complementary pairing and gene expression of sense strand DNA and antisense strand DNA spAAV vectors according to an exemplary embodiment described herein. In this case, spAAV DNA is single polarity, single-stranded DNA. After transfection into the cell, it releases its single polarity, single-stranded DNA. Since this DNA does not have an ITR structure, there is no 3' primer for DNA synthesis, so it cannot synthesize the second complementary DNA strand and therefore cannot express the gene. When another complementary single polarity spAAV virus co-infects, the two complementary single polarity, single-stranded DNA can pair to form a double-stranded DNA and express the gene.

Figure 4 shows a schematic diagram of spAAV vector integration into the cell genome DNA according to an exemplary embodiment described herein. In this case, spAAV DNA is single polarity, single-stranded DNA. After transfection into the cell, it releases its single polarity, single-stranded DNA. This single-stranded DNA may undergo homologous or non-homologous recombination with the cell genome DNA, thereby integrating into the cell genome, then synthesizing the second complementary DNA strand, and potentially expressing the gene.

In another aspect, provided herein is an isolated host cell comprising the spAAV vector described herein.

In some embodiments, the host cells can be eukaryotic cells. In some embodiments, the host cells can be animal cells. In some embodiments, the host cells can be vertebrate cells. In some embodiments, the host cells can be mammalian cells. In some embodiments, the host cells can be human cells.

In some embodiments, the host cells are obtained by contacting eukaryotic cells (e.g., animal cells, e.g., mammalian cells, e.g., human cells) with the spAAV vector described herein.

In another aspect, the spAAV vector described herein is also provided for gene expression. The use of the spAAV described herein in the preparation of products for gene expression is also provided. In some embodiments, the target of gene expression is an animal, specifically a vertebrate, more specifically a mammal, and even more specifically a human.

In another aspect, the spAAV vector described herein is also provided for gene therapy. The use of the spAAV described herein in the preparation of products for gene therapy is also provided. In some embodiments, the target of gene therapy is an animal, specifically a vertebrate, more specifically a mammal, and even more specifically a human.

In another aspect, the spAAV vector described herein is also provided for gene editing. The use of the spAAV described herein in the preparation of products for gene editing is also provided. In some embodiments, the target of gene editing is an animal, specifically a vertebrate, more specifically a mammal, and even more specifically a human.

In another aspect, the spAAV vector described herein is also provided for gene regulation. The use of the spAAV described herein in the preparation of products for gene regulation is also provided. In some embodiments, the target of gene regulation is an animal, specifically a vertebrate, more specifically a mammal, and even more specifically a human.

### Examples

### Construction of Plasmid Vectors for spAAV Vector Packaging

rAAV transgenic plasmids containing ITR-related sequences at only one end of the exogenous gene coding expression frame are also referred to herein as "single polarity" plasmids.

The main plasmid involved here is the spAAV DNA genomic plasmid.

### Construction Method:

### 1. spAAV-EGFP(+) DNA Genomic Plasmid

The plasmid DNA in the 5' to 3' direction contained the D sequence, A'B' sequence, and the DA'B' sequence is: 5'-AGGAACCCCTAGTGATGGAGTTGGCCACTCCCTCTCTGCGCGCTCGCTCG CTCACTGAGGCCGGGCGACCAAA-3' (SEQ ID NO: 5). It included a 1.3 kb stuff1 filler sequence, a forward EGFP gene expression frame (CMV-EGFP-polyA) sequence, and a 1.3 kb stuff2 filler sequence, with the entire packaging frame being 5.0 kb. The EGFP gene expression frame sequence included a CMV promoter, enhancer, intron, EGFP open reading frame, and polyA sequence. The construction used the pFastBacdual-ITR-EGFP as the vector.

First step: construction of pFastBacdual-DtrsA'B'-CMV-EGFP-polyA-Rhstuff. The insertion of **DtrsA'B'.** Primers (primer 1: 5'-CAGGAACCCCTAGTGATGGAGTTGGCCACTCCCTCTCTGCGCGCTCGCTC GCTCACTGAGGCCGGGCGACCAAAA-3' (SEQ ID NO: 6); primer 2: 5'-CGCGTTTTGGTCGCCCGGCCTCAGTGAGCGAGCGAGCGCGCAGAGAGGG AGTGGCCAACTCCATCACTAGGGGTTCCTGGTAC-3' (SEQ ID NO: 7)) were synthesized by Sangon Biotech (Shanghai) Co., Ltd. Denaturation and renaturation were performed. 5 µl of each oligonucleotide (primer 1 and primer 2, each at a concentration of 200 µM), 4 µl of 5× annealing buffer, and deionized water (free of DNAse and RNAse) were added to a final volume of 20 µl. The mixture was reacted at 95°C for 4 minutes in a PCR instrument for annealing. The reaction tube was then removed and placed on the laboratory bench, allowing it to cool to room temperature for 5-10 minutes. This process formed oligo-dsDNA with KpnI/MluI sticky ends, which were inserted into the KpnI/MluI double-digested pFastBacdual-ITR-EGFP vector.

The insersion of **RHstuff**. Primers (primer 3: 5'-GACCGATAATCTCTAGATTGAGGAACAA-3' (SEQ ID NO: 8); primer 4: 5'-AGCTTTGTTCCTCAATCTAGAGATTATCG-3' (SEQ ID NO: 9)) were synthesized by Sangon Biotech (Shanghai) Co., Ltd. Using the same method for denaturation and renaturation, oligo-dsDNA with RsrII/HindIII sticky ends was formed for insertion into the KpnI/MluI double-digested pFastBacdual-DtrsA'B'-CMV-EGFP-polyA vector.

Second Step: Construction of pFastBacdual-DtrsA'B'-1.3kb stuff1-CMV-EGFP-polyA-1.3kb stuff2. 1.3kb stuff1 fragment was cloned by fusing it with the CMV fragment via PCR to create the 1.3kb stuff1-CMV fragment. The primer sequences (primer 5: 5'-TCACTGACGCGTCGGAAGGTCGGTATCCACGGA-3' (SEQ ID NO: 10); primer 6: 5'-CTTCAACCGGCTGAAGCTGGCTAGTTATTAATAGTAATCA-3' (SEQ ID NO: 11); primer 7: 5'-TGATTACTATTAATAACTAGCCAGCTTCAGCCGGTTGAAG-3' (SEQ ID NO: 12); primer 8: 5'-CCCGGGGAATTCAATCGATGTTCGAATCCCAAT-3' (SEQ ID NO: 13)) were synthesized by Sangon Biotech (Shanghai) Co., Ltd. The denatured fragment was inserted into the pFastBacdual-DtrsA'B'-CMV-EGFP-polyA-RHstuff vector using MluI/EcoRI double digestion.

The 1.3kb stuff2 fragment was cloned via PCR. The primer sequences (primer 9: 5'-TTTGTAGGTAACCTCAAGAAGCACATCCTGAATC-3' (SEQ ID NO: 14); primer 10: 5'-GCACGTGGTTACCTTGCCCAGAATGTCTGTGCTG-3' (SEQ ID NO: 15)) were synthesized by Sangon Biotech (Shanghai) Co., Ltd. The denatured fragment was inserted into the pFastBacdual-DtrsA'B'-1.3kb stuff1-CMV-EGF-polyA-RHstuff vector using BstEII single digestion. This resulted in the pFastBacdual-DtrsA'B'-1.3kb stuff 1-CMV-EGF-polyA-1.3kb stuff2 (spAAV-CMV-EGFP(+)) vector, thus obtaining the spAAV-CMV-EGFP(+) genomic packaging plasmid. The construction of the EGFP expression frame can refer to the literature: "Construction of pFastBacdual-ITR-EGFP plasmid" in 1.2.1 method in Li Taiming et al., "Preparation of AAV-ITR gene expression microcarriers in insect cells," Journal of Biotechnology, 2015, 31(8), 1232.

### 2. spAAV-EGFP(-) DNA Genomic Plasmid

The plasmid DNA in the 5' to 3' direction comprises the D sequence, A'B' sequence, and the DA'B' sequence is: 5'-AGGAACCCCTAGTGATGGAGTTGGCCACTCCCTCTCTGCGCGCTCGCTCG CTCACTGAGGCCGGGCGACCAAA-3' (SEQ ID NO: 5). It includes the antisense EGFP gene expression frame sequence, i.e., the polyA sequence, the reverse complementary sequence of the EGFP open reading frame, intron, enhancer, promoter, and downstream Rep recognition and cleavage sequence. The antisense EGFP expression frame was cloned by PCR using pFastBacdual-DtrsA'B'-1.3kb stuff1-CMV-EGF-polyA-1.3kb stuff2 as a template to form the 1.3kb stuff2'-polyA'-EGFP'-CMV'-1.3kb stuff1' fragment. The primer sequences (primer 11: 5'-TCACTGACGCGTCACGTGGTTACCTTGCCCAGA-3' (SEQ ID NO: 16); primer 12: 5'-CAAGATCGGTCCGCGGAAGGTCGGTATCCACGGA-3' (SEQ ID NO: 17)) were synthesized by Sangon Biotech (Shanghai) Co., Ltd. The denatured fragment was inserted into the pFastBacdual-DtrsA'B'-CMV-EGFP-polyA-RHstuff vector using MluI/RsrII double digestion, thereby obtaining the pFastBacdual-DtrsA'B'-1.3kb stuff2'-polyA'-EGFP'-CMV'-1.3kb stuff1' (spAAV-EGFP(-)) genomic packaging plasmid.

### Preparation of Recombinant Bacmid

Recombinant Bacmids were prepared from the three recombinant plasmids obtained in the previous step. The specific process is as follows:
1. 100 µl of DH10Bac competent cells were slowly thawed on ice.
2. 500 ng of plasmid DNA was added and gently mixed.
3. The mixture was placed on ice for 30 minutes, heat-shocked at 42°C for 90 seconds, and immediately returned to ice for 2 minutes.
4. 900 µl of LB medium was added and shaken at 225 rpm at 37°C for 2-3 hours.
5. On pre-prepared 90 mm agar plates containing 50 µg/ml Kan, 7 µg/ml Gen, and 10 µg/ml Tet, 40 µl of 2% (20 mg/ml) X-gal and 20 µl of 20% (200 mg/ml) IPTG were added to the center. A sterile spreader was used to disperse the solution over the entire surface of the culture plate, and it was incubated at room temperature until all the liquid had evaporated.
6. The cells were diluted in LB medium using a 10-fold gradient (10⁻¹, 10⁻²). 100 µl from each gradient was spread on LB plates.
7. The plates were incubated at 37°C for 48 hours. Two white colonies were picked and streaked onto new LB agar plates (with the same antibiotics) and incubated overnight at 37°C.
8. Two single colonies were picked for PCR identification. Bacmid is identified by PCR, wherein the primers used were target gene F/R and PUC M13 F/R.
9. The correctly identified Bacmid colonies were inoculated into 10 ml LB (Kan+, Gen+, Tet+) and cultured with shaking for 12 hours. Recombinant Bacmid DNA was extracted using an OMEGA kit following the manufacturer's instructions. After measuring the Bacmid DNA concentration, it was aliquoted and stored at - 20°C.

### Preparation of Baculovirus

### 1. Preparation of Transfection Reagents

### 1×HBS (200ml) Preparation:

| | |
|---|---|
| Hepes: | 0.954 g |
| NaCl: | 1.754 g |
| Sterilized ddH₂O: | 150 ml |
| Use 1M NaOH to adjust pH to 7.4 | |

Make up to 200ml with sterilized ddH₂O, filter sterilize in a biosafety cabinet, store at 4°C.

| | |
|---|---|
| PEI (20ml) Preparation: | |
| PEI: | 0.043 g |
| Absolute ethanol: | 1 ml |

Dissolve thoroughly, then make up to 20 ml with 1×HBS, freeze-thaw three times (-20°C to room temperature), store at -20°C.

### 2. Cell Seeding

Seeding: A 6-well plate was taken, and suspension cultured cells were counted to achieve a cell density of 2*10⁶ cells/ml. 2 ml of the cell suspension was added to each well, ensuring a cell viability above 95%.

### 3. Transfection (per well)

Solution A: 6 µl PEI and 94 µl 1×HBS were added, mixed well, and allowed to stand for 4 minutes.

Solution B: 3 µg of Bacmid DNA (pre-inactivated at 65°C for 30 minutes) was taken, and the volume was made up to 100 µl with 1×HBS, gently mixed.

100 µl of Solution A was added to Solution B, mixed well, and incubated at room temperature for 30 minutes. The mixture was then added to the seeded cells and incubated for 96 hours.

### 4. Virus Amplification

### 1) Isolation of P1:

After confirming the cells were in the late infection stage (96 h), 2 ml of virus-containing medium from each well was collected into a sterile 15 ml centrifuge tube. The tube was centrifuged at 500 g for 5 minutes to remove cell debris.

The supernatant was transferred to a sterile EP tube and stored at 4°C in the dark. For long-term storage, the supernatant was aliquoted and frozen at -80°C.

### 2) Amplification to obtain P2:

Cells with an MOI of 0.1 were taken, 8 ml of suspension culture with a density of 2×10^6 cells/ml. The required P1 volume was calculated to be 1.6 ml. The cells were incubated at 27°C for 72 hours, then the suspension culture cells were collected into a sterile 15 ml centrifuge tube and centrifuged at 500g for 5 minutes. The supernatant was aliquoted into sterile EP tubes. This virus supernatant was P2, stored at 4°C in the dark. For long-term storage, it was aliquoted and frozen at -80°C.

### 3) Amplification to obtain P3:

Following the above method, P3 was amplified. Cells with an MOI of 0.1, a density of 2×10⁶ cells/ml, and 10 ml of suspension culture were taken, and 200 µl of P2 storage solution was added. The cells were incubated for 72 hours to collect P3.

Typically, the obtained P1 virus titer ranged between 1×10⁶ and 1×10⁷, and the P2 titer ranged between 1×10⁷ and 1×10⁸.

### 4) Virus packaging:

Cells with an MOI of 1, 100 ml of suspension culture with a density of 5×10⁶ cells/ml, were taken. 5 ml each of P3 Rep, Cap, and target gene were added, and the cells were incubated for 72 hours to collect the cells.

### spAAV Vector Packaging

The spAAV vector packaging system described herein can be an insect cell baculovirus packaging system or a mammalian cell packaging system. The insect cells used were Sf9 cells. First, one or two recombinant plasmids expressing the AAV replication protein Rep and the AAV capsid protein Cap (with Rep and Cap genes either on one plasmid or on two different plasmids), and another plasmid containing the spAAV DNA genome, were respectively transformed into E. coli DH10Bac competent cells. Through two rounds of blue-white screening, recombinant bacmid-containing colonies appeared white, while non-recombinant colonies appeared blue. White colonies were picked for amplification, and recombinant bacmid was extracted.

Next, using insect cell transfection reagents, the above two or three types of recombinant bacmids were respectively transfected into Sf9 insect cells. After 4-5 days, the cell supernatant was collected to obtain P1 generation recombinant baculovirus from insect cells. The P1 generation recombinant baculovirus was then amplified by infecting Sf9 insect cells twice to obtain P3 generation recombinant baculovirus. The titer of the P3 generation baculovirus was determined using the plaque assay, where virus titer (pfu/ml) = 1/dilution factor × number of plaques × 1/inoculation volume per well.

At last, the P3 generation recombinant baculovirus (two or three types) was co-infected into Sf9 insect cells to package and obtain spAAV vector virus particles. This operation can refer to the literature: Urabe M, Ding C, Kotin RM. Insect cells as a factory to produce adeno-associated virus type 2 vectors. Hum Gene Ther. 2002 Nov 1;13(16):1935-43. doi: 10.1089/10430340260355347. PMID: 12427305.

A schematic diagram of the insect cell baculovirus packaging system is shown in Figure 2.

### Iodixanol Density Gradient Ultracentrifugation Purification Steps

### 1. Obtaining Cell Lysate:

1) After 72 hours of co-transfection, the cells were collected by centrifugation at 1000g for 5 minutes.
2) The supernatant was discarded, and the cells were resuspended in 10 ml of Lysis Buffer.

### Lysis Buffer (1L):

| | |
|---|---|
| NaCl: | 8.766g |
| Tris: | 6.055g |
| DH₂O: | 950ml |

Use 5M HCl to adjust pH to 8.5, make up to 1L

Sterile filtered in a biosafety cabinet, stored at 4°C

3) The suspension was frozen in liquid nitrogen and thawed at 37°C, repeating 3-4 times until clear.

4) The lysate was centrifuged at 5000g for 30 minutes at 4°C to collect the supernatant; the pellet was resuspended in 4-5 ml PBS and centrifuged again to collect the supernatant.

5) DNase (10µl/ml) and RNase (1µl/ml) were added and digested in a 37°C water bath for 1 hour before purification.

### 2. Iodixanol Density Gradient Ultracentrifugation Purification of Virus:

1) Using PBS-MK (1×PBS, 1mM MgCl2, 2.5mM KCl), 60% iodixanol was diluted to 15%, 25%, 40%, and 58%. Solid NaCl was added to the 15% iodixanol to a final concentration of 1M.
2) Phenol red solution (0.5%) was added to the 15% and 25% iodixanol to a final concentration of 1.5gl/ml, and to the 58% iodixanol to a final concentration of 0.5µl/ml.
3) Using a 1.27*120mm blunt spinal needle, 8ml of 15%, 6ml of 25%, 8ml of 40%, and 5ml of 58% iodixanol solutions were sequentially added to a 39ml quick-seal tube from the bottom, avoiding bubbles.
4) The cell lysate was carefully layered on top of the iodixanol density gradient solution to fill the tube (flush with the tube opening). If necessary, Lysis Buffer was used to top off the volume. The tube was balanced and centrifuged at 69000 rpm, 18°C for 1.5 hours (BECKMAN COULTER centrifuge, rotor 70Ti).
5) The quick-seal tube was punctured from the bottom, discarding the first 4ml (corresponding to the 58% layer), and 6ml of the solution in the tube was collected.
6) The collected solution was ultrafiltered using Amicon Ultra-4 Centrifugal Filters, Ultracel-100k, centrifuged at 3000g for 5 minutes. PBS (1×PBS, Solarbio, supplemented with 5M NaCl to a final concentration of 350mM) was used to wash repeatedly until the residual iodixanol concentration was less than 0.1%. The final virus solution volume was concentrated to about 200µl per 100ml of packaging volume.
7) Glycerol was added to a final concentration of 5%, sterile filtered, aliquoted, and stored at -80°C.

### Method for Quantitative Fluorescence PCR Detection of spAAV Vector Titer

### RT-PCR Procedure:

1. The plasmid ITR-EGFP was diluted 10 times, followed by a gradient dilution of 5 levels to create a standard curve.
2. The virus was diluted 10 times, followed by a gradient dilution of 4 levels.
3. Preparation of Buffer (protected from light):

| | |
|---|---|
| 2xSuperPreMix Plus (SYBR Green): | 10 µl |

Forward primer, 5'-TCCGCGTTACATAACTTACGG-3' (SEQ ID NO: 18; synthesized by Sangon Biotech (Shanghai) Co., Ltd.): 0.3 µl Reverse primer, 5'-GGGCGTACTTGGCATATGAT-3' (SEQ ID NO: 19; synthesized by Sangon Biotech (Shanghai) Co., Ltd.): 0.3 µl DD H₂O: 4.4 µl
4. Sample loading, 20 µl system: 5 µl template + 15 µl Buffer, each gradient with two replicates.
5. RT-PCR (Roche LightCycler) procedure:

| | | | |
|---|---|---|---|
| Stage 1: | Pre-denaturation | Reps 1 | 95°C for 600s |
| Stage 2: | Cyclic reaction | Reps 40 | 95°C for 10s |
| | | | 55°C for 30s |
| | | | 72°C for 30s |
| Stage 3: | Melting curve | Reps 1 | 95°C for 15s |
| | | | 60°C for 60s |
| | | | 95°C for 15s |

### SDS-PAGE Electrophoresis Analysis of spAAV Vector Capsid Proteins

### 1. Gel Preparation:

1) Separating Gel (10%): In the separating gel preparation beaker, the following components were added (9.093 ml total): double-distilled water: 3.69 ml; 30% Acrylamide solution (acrylamide: methylene-diacrylamide = 29:1): 2.97 ml; 2M Tris, pH 8.8: 2.25 ml; 10% ammonium persulfate: 90 µl; 10% SDS: 90 µl; TEMED: 3 µl. After mixing, the separating gel was pipetted between the two glass plates until the liquid level reached 1 cm below the comb. 75% ethanol was gently added on top with a dropper and left to stand until the separating gel polymerized. The 75% ethanol layer was then discarded.
2) Concentrating Gel (5%): In a beaker for the concentrating gel, the following components were added in sequence (total volume 2.357 ml): 1.83 ml of double-distilled water, 0.39 ml of 30% acrylamide gel solution (acrylamide: bisacrylamide = 29:1), 0.75 ml of 0.5M Tris, pH 6.8, 30 µl of 10% ammonium persulfate, 30 µl of 10% SDS, and 2 µl of TEMED. After mixing, the concentrating gel was immediately pipetted over the separating gel between two glass plates until full, and a comb was gently inserted. After standing still for it to solidify, the gel plate was formed.

### 2. Sample Processing:

Virus Samples: The virus samples were mixed with 5X loading buffer in a 2:1 ratio and heated in a boiling water bath for 10 minutes. After boiling, they were immediately centrifuged, and the entire sample was used for electrophoresis.

### 3. Electrophoresis

1) The gel plate made of two glass plates was vertically mounted on the power rack in the electrophoresis tank, with the grooved side facing the power rack.
2) The gel plate and power rack were secured in the electrophoresis tank as required, electrophoresis buffer was added as needed, and the comb was gently removed from the gel plate.
3) Processed sample solution (15 µl) was taken with a micro-syringe and slowly added to the wells (sample loading site) in the gel plate.
4) During electrophoresis, voltage was controlled with 90V for the concentrating gel and 120V for the separating gel. Electrophoresis was stopped when the bromophenol blue dye in the gel reached about 1 cm from the bottom.

4. Staining: The gel plate was gently pried open with a blade or thin plate, and the separating gel was cut off along the junction of the separating gel and the concentrating gel with a blade. The separating gel was carefully transferred to a staining vessel, 100 ml of staining solution was added, covered, and stained for 1-3 hours.

5. Decolorization: The staining solution was poured away. The stained gel was rinsed several times with water, and put into clean water until the water just covers the gel, and then put into a microwave oven, heated on high heat for 2 minutes, taken out and shaked slowly. This operation was repeated until the protein bands could be clearly seen.

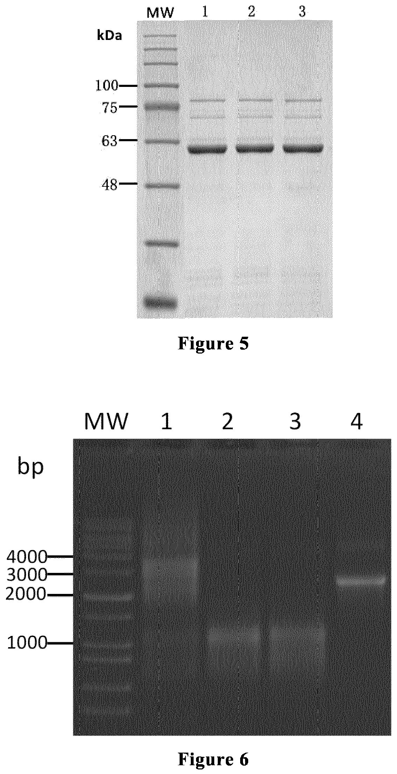

Figure 5 shows the SDS-PAGE electrophoresis results of the spAAV capsid protein. The composition of the spAAV capsid proteins is the same as that of the standard rAAV capsid proteins, comprising VP1, VP2, and VP3 proteins, with the composition ratio of approximately 1:1:10.

### Identification of spAAV Vector Genome

### Neutral Agarose Gel Electrophoresis Analysis of DNA

1. An accurate amount of agarose powder and a measured volume of 1X TAE electrophoresis buffer were added to an Erlenmeyer flask or glass bottle to prepare the agarose solution.
2. A Kimwipes paper was gently placed in the neck of the flask. If a glass bottle was used, the cap was loosened. The suspension was heated in a boiling water bath or microwave until the agarose dissolved.
3. The clear solution was cooled to 40-50°C, and 4S red staining solution was quickly added to cast the gel. Once the gel completely solidified, it was placed in the electrophoresis tank, and freshly prepared 1X TAE electrophoresis buffer was added to just cover the gel.
4. 10 µl of virus sample was prepared, and 1.1 µl of 10X PCR Buffer was added. The mixture was placed in a PCR instrument with the following cycle: 95°C for 5 minutes, 72°C for 5 minutes, 55°C for 5 minutes, 37°C for 5 minutes, 80°C for 5 minutes, 72°C for 5 minutes, 55°C for 5 minutes, and 37°C for 15 minutes.
5. 0.2 volumes of 6X gel loading buffer were added to the denatured samples.
6. The entire sample, dissolved in 6X loading buffer, was added to the sample wells. Electrophoresis was run at 100V for 50 minutes.
7. Imaging: The gel was placed in a gel imaging system for photographing and recording.

Figure 6 shows the neutral agarose gel electrophoresis results of the spAAV genomic vector. spAAV DNA is single polarity single-strand DNA. In neutral agarose gel electrophoresis analysis, the DNA appeared as much smaller DNA bands than its molecular weight and often appeared diffuse.

### DNA Alkaline Agarose Gel Electrophoresis Analysis

1. Preparation of Alkaline Agarose Gel: 0.36g of agarose powder and 27ml of distilled water were added to an Erlenmeyer flask or glass bottle to prepare the agarose solution. A Kimwipes paper was gently placed in the neck of the Erlenmeyer flask. If using a glass bottle, the cap was loosened. The suspension was heated in a microwave on medium heat until the agarose dissolved. The clear solution was equilibrated in a 56°C water bath for 5 minutes. Then, 3ml of 10X alkaline gel electrophoresis buffer (equilibrated in a 56°C water bath for 2 minutes) was added and mixed to quickly cast the gel. Once the gel solidified completely, it was placed in the electrophoresis tank, and freshly prepared 1X electrophoresis buffer (4°C) was added to just cover the gel.
2. Sample Preparation: 30µl of virus sample was taken, and 3µl of proteinase K (20mg/ml) was added. The mixture was digested at 65°C for 15 minutes and centrifuged at 12,000g for 5 minutes. 30µl of the supernatant was taken, and 6µl of 6X alkaline gel loading buffer was added.
3. Electrophoresis: The entire DNA sample was added to the wells, and electrophoresis was started at a voltage of <3.5 V/cm (27V in a horizontal electrophoresis tank JY-SPAT for 3 hours).
4. Elution: The gel was placed in 400ml of water for injection and eluted on a horizontal shaker at 56rpm for 1 hour, the water was changed every 30 minutes.
5. Staining: The eluted gel was stained with 1X TAE staining solution containing 0.5 µg/ml ethidium bromide (EB).
6. Imaging: The gel was photographed and recorded using a gel imaging system.

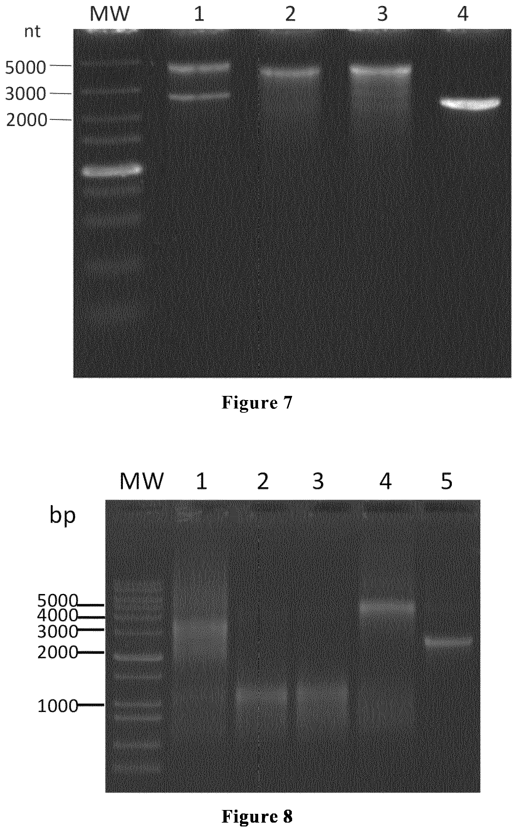

Figure 7 shows the alkaline agarose gel electrophoresis results of the spAAV vector. spAAV DNA is single polarity single-stranded DNA, which appears as a single DNA band with a molecular weight of 4.7kb in alkaline agarose gel electrophoresis analysis. Traditional rAAV EGFP packaging often results in DNA molecules with different molecular weights, this rAAV EGFP packaging produces DNA molecules with two molecular weights, including single-stranded DNA molecules of 4.7kb and 2.7kb.

### Annealing Identification of Single Polarity Genome Containing Sense and Antisense Strands of Target Gene DNA

8µl each of spAAV-EGFP(+) DNA and spAAV-EGFP(-) DNA were mixed with 4 µl of 5× annealing buffer. The mixture was incubated at 95°C for 15 minutes, cooled to room temperature on the lab bench, and then incubated at 37°C in a water bath for 30 minutes. DNA was subjected to neutral agarose gel electrophoresis, followed by Goldview staining analysis.

Figure 8 shows the neutral agarose gel electrophoresis results of the complementary pairing of sense and antisense strands of spAAV DNA. spAAV DNA is single polarity single-stranded DNA, which appears as much smaller DNA bands in neutral agarose gel electrophoresis analysis and often appears diffuse. If the sense strand DNA of the spAAV vector and the antisense strand DNA of the spAAV vector are annealed together, their sense and antisense DNA molecules will pair complementarily to form double-stranded DNA, with a molecular weight of 4.7kb.

### S1 Nuclease Digestion for Identifying spAAV Vector Single-Stranded DNA Genome

S1 nuclease and buffer, 5X Reaction Buffer: S1 Nuclease = 10: 1, were mixed and added to the extracted spAAV vector DNA genome. The mixture was incubated at 37°C for 30 minutes, followed by DNA neutral agarose gel electrophoresis for detection.

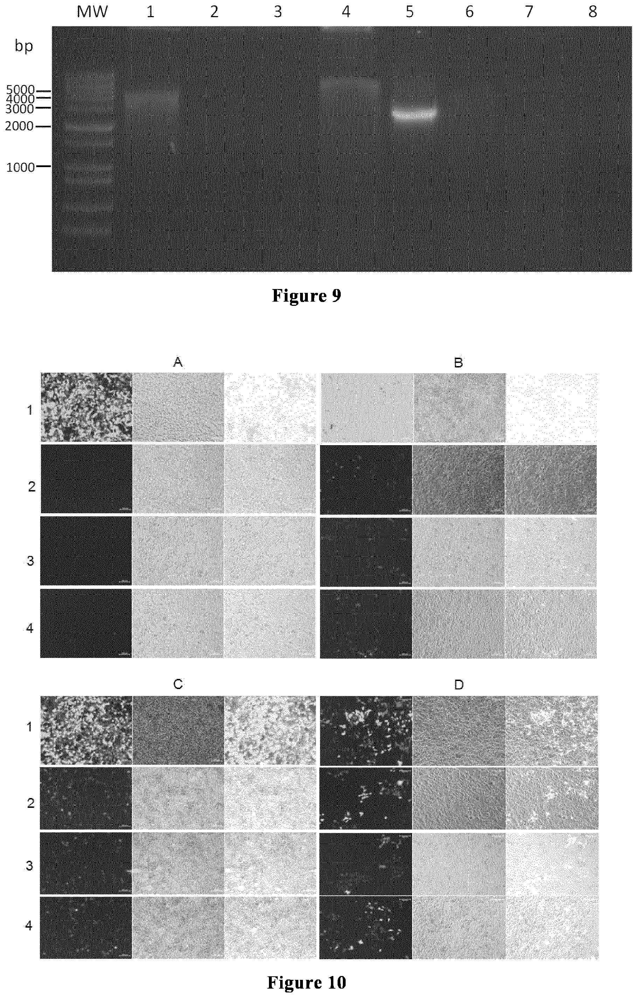

Figure 9 shows the neutral agarose gel electrophoresis results of spAAV DNA after S1 nuclease digestion. S1 nuclease is a specific single-strand nucleotide endonuclease that degrades single-stranded DNA. Since spAAV DNA is single polarity, single-stranded DNA, it was degraded by S1 nuclease. However, when the sense strand and antisense strand DNA molecules of spAAV anneal together, they form double-stranded DNA, which is not degraded by S1 nuclease.

### Gene Expression of spAAV Vector in HEK293 Cells

HEK293 cells were seeded in a 24-well plate at a cell density of 1.5×10⁵. The following day, the cells were transfected with rAAV6-EGFP, spAAV-EGFP(+), and spAAV-EGFP(-), respectively. HEK293 cells were co-infected with spAAV-EGFP(+) and spAAV-EGFP(-) at an MOI of 1×10⁶. Fluorescence microscopy was used to observe and photograph the fluorescence of the cells.

Figure 10 shows the gene expression results in HEK293 cells infected with the spAAV vector. Since the spAAV genomic DNA is single-stranded, lacks an ITR structure, and has no 3' primer for DNA synthesis, it cannot synthesize a second complementary DNA strand, and it cannot synthesize a second complementary DNA strand in cells. Thus, it cannot form a double-stranded DNA like the bipartite rAAV vector, which can express genes by pairing complementary sense and antisense DNA molecules. As a result, almost no fluorescent protein expression was observed in the first two days after infection with spAAV. A few cells gradually exhibited fluorescent protein expression three days after infection, and after about a week, more cells expressed the fluorescent protein. These fluorescent cells appeared as adjacent clusters, and the fluorescent protein expression was stable and did not diminish with cell division. This expression pattern is due to the integration of the single polarity, single-stranded DNA released by the spAAV vector into the cell genome through homologous or non-homologous recombination, leading to stable and long-term gene expression.

When HEK293 cells were simultaneously infected with sense strand DNA spAAV vector and antisense strand DNA spAAV vector, the released sense and antisense DNA molecules could pair to form double-stranded DNA, enabling gene expression. Protein expression through complementary pairing of spAAV vectors is generally faster and more efficient.

Thus, it can be seen that when cells cultured *in vitro* (e.g., HEK293 cells) are infected separately with either sense strand or antisense strand DNA-containing spAAV described herein, i.e., spAAV-EGFP(+) or spAAV-EGFP(-), the genomic DNA can integrate into the cell genome, producing long-term and stable fluorescent protein expression. However, when cells cultured *in vitro* (e.g., HEK293 cells) are co-infected with both sense strand and antisense strand DNA-containing spAAV vectors described herein, i.e., spAAV-EGFP(+) and spAAV-EGFP(-), the two can complement each other to produce double-stranded DNA molecules, resulting in a higher proportion of target gene expression.

## Claims

1. An unpackaged single polarity recombinant adeno-associated virus (spAAV) genome, having linear, open-ended termini at both ends, with one end lacking an inverted terminal repeat (ITR) or a portion thereof, and the other end having a truncated ITR (ITRt), wherein the ITRt does not form a T-shaped hairpin palindrome structure; specifically, the spAAV genome is single polarity, single-stranded DNA.

2. The unpackaged spAAV genome according to claim 1, wherein the 3' end of the unpackaged spAAV genome lacks an ITR or a portion thereof, and the 5' end comprises the ITRt.

3. The unpackaged spAAV genome according to claim 1, wherein the ITRt comprises a Rep protein binding element (RBE) and a terminal resolution site (trs).

4. An spAAV vector, comprising:
a capsid protein; and
an spAAV genome packaged by the capsid protein;
wherein the spAAV genome packaged by the capsid protein is derived from the unpackaged spAAV genome according to claim 1.

5. The spAAV vector according to claim 4, wherein the spAAV genome packaged by the capsid protein is single polarity, single-stranded DNA.

6. An spAAV transgenic plasmid for producing the unpackaged spAAV genome according to claim 1.

7. A method of delivering an exogenous nucleic acid to a cell, comprising contacting the cell with one or more spAAV vectors according to claim 4, wherein the genome of the one or more spAAV vectors comprises a gene expression cassette of the exogenous nucleic acid and/or optionally other DNA sequences.

8. The method according to claim 7, wherein the one or more spAAV vectors comprise an spAAV vector containing only the sense strand single-stranded DNA sequence of the exogenous nucleic acid and/or an spAAV vector containing only the antisense strand single-stranded DNA sequence of the exogenous nucleic acid.

9. An isolated host cell comprising the spAAV vector according to claim 4.

10. Use of the spAAV vector according to claim 4 in the preparation of a product for gene expression, gene therapy, gene editing, or gene regulation.
